# EUROPÄISCHE PATENTSCHRIFT

(11) **EP 0 614 462 B1**
(45) Veröffentlichungstag und Bekanntmachung des Hinweises auf die Patenterteilung: **29.12.1997**
(21) Anmeldenummer: 92924601.5
(22) Anmeldetag: 23.11.1992
(51) Int. Cl.: C07H 19/167, C07H 19/20, C07H 19/173, C07H 19/19, C07D 473/00

(54) **NEUES VERFAHREN ZUR HERSTELLUNG VON 2-FLUORPURIN-DERIVATEN**
NEW METHOD OF PREPARING 2-FLUOROPURINE DERIVATIVES
NOUVEAU PROCEDE DE FABRICATION DE DERIVES DE LA 2-FLUOROPURINE

(30) Priorität: 25.11.1991 DE 4139238; 12.12.1991 DE 4141454
(43) Veröffentlichungstag der Anmeldung: 14.09.1994
(73) Patentinhaber: SCHERING AKTIENGESELLSCHAFT, 13353 Berlin (DE)
(72) Erfinder: VORBRÜGGEN, Helmut, D-1000 Berlin 27 (DE); KROLIKIEWICZ, Konrad, D-1000 Berlin 47 (DE); WIRSCHING, Randolph, C., Livermore, CA 94550 (US); BAUMAN, John, G., Alameda, CA 94501 (US)
(86) Internationale Anmeldenummer: EP9202689
(87) Internationale Veröffentlichungsnummer: WO9311144

(56) Entgegenhaltungen:
- WO-A-90/14352
- CHEMICAL ABSTRACTS, vol. 99, 1983, Columbus, Ohio, US; abstract no. 140313, I.MATSUMOTO '2-Fluoro-6-methylpurine Riboside.' Seite 634 ;Spalte 2 ;

## Beschreibung

Die Erfindung betrifft eine neue Darstellung von 2-Fluorpurinnucleosiden und ihre 5'-Phosphate sowie ihre Verwendung zur Darstellung von pharmazeutischen Produkten.

Nach Arbeiten von J. Montgomery und Mitarbeitern läßt sich freies 2-Aminoadenosin in einer Schiemann-Reaktion mit wässriger Fluorborsäure in 2-Fluoradenosin überführen (J. Montgomery + K. Hewson, J.A.C.S. **82**, 463 (1960)). Dieses Verfahren ist jedoch für präparative Zwecke ungeeignet, da die Ausbeuten unter 10% liegen.

Es ist in der Literatur ferner bekannt, daß man 2-Aminoadenosin-2',3',5'-tri-O-acetat mit tert.-Butylnitrit oder Natriumnitrit in 50 % HF/Pyridin in ein Gemisch des entsprechenden 2-Fluoradenosin-2',3',5'-tri-O-acetats und 2,6-Difluor-nebularin-2',3',5'-tri-O-acetats umwandeln kann, das mit trockenem Ammoniakgas in 1,2-Dimethoxyäthan reines 2-Fluoradenosin-2',3',5'-tri-O-acetat in ca. 60 % Ausbeute liefert (M.J. Robins, B. Uznanski, Can. J. Chem. **59**, 2608 (1961)). Ein Nachteil dieses Verfahrens ist jedoch, daß wegen des erhaltenen Gemisches ein weiterer Reaktionsschritt notwendige ist, um das reine 2-Fluornucleosid zu erhalten.

Da sich 2-Aminoadenosin aber nicht selektiv zum 2-Aminoadenosin-2',3',5'-tri-O-acetat acetylieren läßt (vergl. u.a. J.A. Montgomery und K. Hewson, J.Med. Chem. **12**, 498 (1969)) - man erhält statt des Tri-O-acetates ein kristallines Gemisch von hauptsächlich Adenosin-N²-2',3',5'-O-tetraacetat neben wenig Pentaacetat -, ist das für die Durchführung der Schiemann-Reaktion in 50 % HF-Pyridin nach M.J. Robins und B. Uznanski, Can. J. Chem. **59**, 2608 (1961) notwendige 2-Aminoadenosin-2',3',5'-tri-O-acetat nur auf Umwegen und unter großem Aufwand zugänglich (M.J. Robins, B. Urbanski, Can. J. Chem. **59,** 2601 (1981)).

Aus der WO 90/14352 ist die Herstellung von 2-Halo-9-(2-deoxy-2-fluor-B-D-arabinofuranosyl)-adeninnucleosiden bekannt, wobei die Umsetzung zu einer Ausbeute von bis zu 70% führt. Es ist ferner bekannt, daß 6-Methylpurine durch Umsetzung mit Tetrafluoroborsäure eine Ausbeute von 55,1 % ergeben (Chemical Abstracts Vol. 99, 1983, Seite 634, 140313y).

Zusammenfassend läßt sich feststellen, daß bei allen bekannten Verfahren die 2-Fluorierung unter Verwendung von O-geschützten Nucleosiden oder Nucleotiden durchgeführt wird oder zu geringeren Ausbeuten an Verfahrensprodukt führt.

Es war deshalb überraschend, daß sich ungeschützte Verbindungen der Formel II 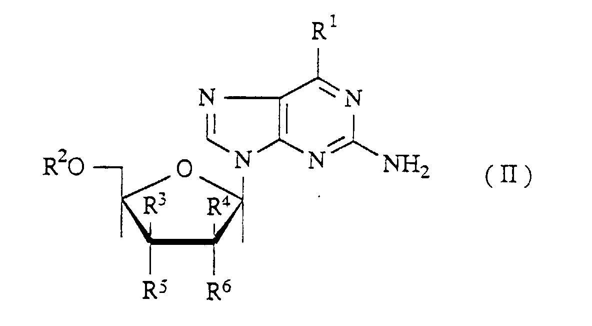 in Ausbeuten von mehr als 90% in 50% HF/Pyridin oder HF/Pyridin/H₂O zu den entsprechenden 2-Fluorpurin-Derivaten der Formel I, 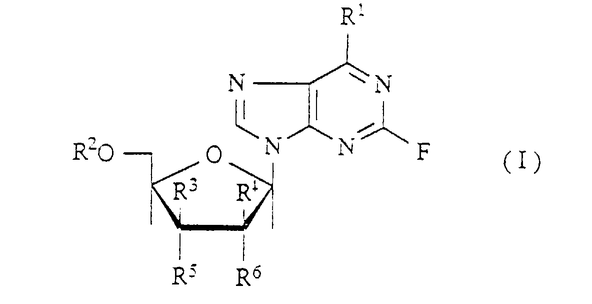 umsetzen lassen.

Die vorliegende Erfindung betrifft daher ein neues Verfahren zur Herstellung von 2-Fluorpurin-Derivaten der allgemeinen Formel I 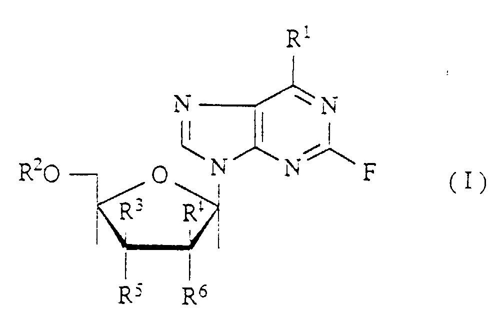 worin
R¹ OH, NH₂, NR⁷R⁸ oder NR⁹R¹⁰,
R² Wasserstoff oder 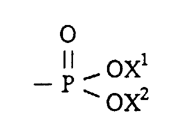 bedeuten,
   wobei
X¹,X² unabhängig voneinander Wasserstoff, Li, Na, K oder Benzyl darstellen,
R³,R⁴,R⁵,R⁶ unabhängig voneinander Wasserstoff oder OH,
R⁷,R⁸ unabhängig voneinander Wasserstoff, C₁-C₄ Alkyl, C₇-C₈ Aralkyl, einen heterocyclischen Rest, C₃-C₇ Cycloalkyl, oder R⁷ und R⁸ gemeinsam einen C₄-C₇ Ring, der gegebenenfalls 1-3 weitere Heteroatome enthält, darstellen, oder wenn R⁷ Wasserstoff bedeutet, R⁸ OH oder NH₂ darstellen kann,
R⁹ Wasserstoff oder C₁-C₄ Alkyl,
R¹⁰ -(CH₂)ₙ-R¹¹, 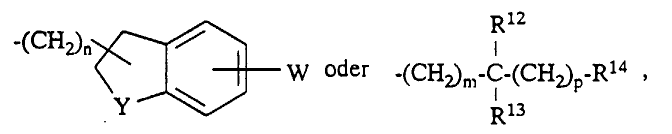
m,n,p unabhängig voneinander 0,1 oder 2,
R¹¹ gegebenenfalls durch C₁-C₄ Alkyl substituierte Reste C₃-C₇ Cycloalkyl, Cyclohexenyl, Bicycloheptyl oder Bicycloheptenyl,
Y Stickstoff, Sauerstoff, Schwefel, Methylen, -CH₂Z- oder -ZCH₂-,
wobei Z Stickstoff, Sauerstoff, oder Schwefel darstellt,
R¹²,R¹³ unabhängig voneinander Wasserstoff, OH, Phenyl oder C₁-C₄ Alkyl,
R¹⁴ Wasserstoff, OH, C₁-C₄ Alkyl, Phenyl oder gegebenenfalls durch ein bis drei der folgenden Reste C₁-C₄ Alkyl, C₁-C₄ Alkoxy, OH, C₁-C₄ Alkanoyloxy, Benzyloxy, Trifluormethyl, oder Halogen substituiertes Phenyl,
W Wasserstoff, C₁-C₄ Alkoxy, C₁-C₄Alkylthio, Halogen oder gegebenenfalls durch OH, C₁-C₄Alkoxy, CN, COOR¹⁵ oder CONHR¹⁶ substituiertes C₁-C₄ Alkyl,
R¹⁵ Wasserstoff oder C₁-C₄ Alkyl,
R¹⁶ Wasserstoff oder C₁-C₄ Alkyl,
bedeuten,
dadurch gekennzeichnet, daß ein 2-Aminopurin-Derivat der allgemeinen Formel II 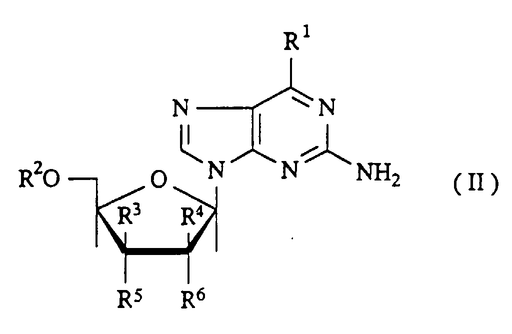 worin R¹, R², R³, R⁴, R⁵ und R⁶ die oben angegebenen Bedeutungen haben, in HF/Pyridin oder HF/Pyridin/H₂O in Gegenwart von einem Nitrit der allgemeinen Formel III,

R¹⁷ONO (III),

worin R¹⁷ vorzugsweise tert.-Butyl, Li, Na, oder K bedeutet,
umgesetzt wird und gegebenenfalls, wenn R² Wasserstoff bedeutet, nachträglich in das Phosphat überführt wird.

Von den Resten R³,R⁴,R⁵,R⁶ sind vorzugsweise mindestens zwei Reste Wasserstoff und maximal zwei Reste Hydroxy, wobei niemals zwei Hydroxyreste an demselben Kohlenstoffatom stehen dürfen.

Die Alkylreste in R⁷, R⁸, R⁹, R¹², R¹³, R¹⁴, R¹⁵ und R¹⁶ können Methyl, Ethyl, Propyl, Isopropyl, Butyl, Isobutyl, sek.-Butyl und tert.-Butyl sein.

Als Alkylreste für W kommen unsubstituierte Reste wie Methyl, Ethyl, Propyl, Isopropyl, Butyl, Isobutyl sek.-Butyl und tert.-Butyl sowie durch Hydroxy, C₁-C₄-Alkoxy, CN, COOR¹⁵, oder CONHR¹⁶ substituierte C₁-C₄ Alkylreste in Frage wie zum Beispiel Hydroxymethyl, Hydroxyethyl, Hydroxypropyl, Hydroxybutyl, Methoxymethyl, Methoxyethyl, Methoxypropyl, Methoxybutyl, Ethoxymethyl, Ethoxyethyl, Ethoxypropyl, Ethoxybutyl, Cyano-methyl, Cyanoethyl, Cyanopropyl, Cyanobutyl, Carboxy-methyl, Carboxyethyl, Carboxypropyl, Carboxybutyl, Methoxy-carbonylmethyl, Ethoxycarbonylmethyl, Methoxy-carbonylethyl, Ethoxy-carbonylethyl, Carbamoyl, Carbamoylmethyl, Carbamoylethyl, Carbamoylpropyl, Carbamoylbutyl, N-Methylcarbamoylmethyl, N-Methyl-carbamoylethyl.

Als Aralkylreste R⁷ und R⁸ sind Benzyl Phenethyl und 1-Phenylethyl gemeint.

Für den heterocyclischen Rest in R⁷ und R⁸ kommen 2-Pyridyl, 3-Pyridyl, 4-Pyridyl, 2-Furyl, 3-Furyl, 2-Thienyl, 3-Thienyl, 2-Pyrrolyl, 3-Pyrrolyl 2-Indolyl und 3-Indolyl in Frage, die gegebenenfalls durch Fluor, Chlor, Brom, Cyano, Hydroxy, Carboxy, C₁-C₄ Alkoxy oder C₁-C₄ Alkyl substituiert sein können.

Die Cycloalkylreste in R⁷ und R⁸ sind Cyclopropyl, Cyclobutyl, Cyclopentyl, Cyclohexyl und Cycloheptyl, wobei Cyclopentyl, Cyclohexyl und Cycloheptyl durch Cyano, Hydroxy, Methyl, Ethyl,Propyl, Butyl, Methoxy, Ethoxy, Carboxy, Methoxycarbonyl oder Ethoxycarbonyl substituiert sein können.

Die Cycloalkylreste in R¹¹ sind Cyclopropyl, Cyclobutyl, Cyclopentyl, Cyclohexyl und Cycloheptyl, wobei Cyclopentyl, Cyclohexyl und Cycloheptyl durch Methyl, Ethyl, Propyl, Isopropyl, Butyl, Isobutyl, sek.-Butyl oder tert.-Butyl substituiert sein können.

Die Cyclohexenylgruppe in R¹¹ kann gegebenenfalls durch Methyl, Ethyl, Propyl, Isopropyl, Butyl, Isobutyl, sek.-Butyl oder tert.-Butyl substituiert sein.

Mit der Bicycloheptylgruppe in R¹¹ ist eine [2.2.1]Bicycloheptyl-gruppe gemeint. Diese kann gegebenenfalls durch Methyl, Ethyl, Propyl, Isopropyl, Butyl, Isobutyl, sek.-Butyl oder tert.-Butyl substituiert sein.

Mit dem Bicycloheptenylrest in R¹¹ ist ein [2.2.1]Bicycloheptenyl-rest gemeint, der gegebenenfalls durch Methyl, Ethyl, Propyl, Isopropyl, Butyl, Isobutyl, sek.-Butyl oder tert.-Butyl substituiert sein kann.

Wenn R⁷ und R⁸ gemeinsam einen C₄-C₇-Ring darstellen, sind damit Reste wie zum Beispiel Pyrrolidin, Piperidin oder Morpholin gemeint.

Als substituierte Phenylreste in R¹⁴ kommen Methylphenyl, Ethylphenyl, Propylphenyl, Butylphenyl, Dimethylphenyl, Methoxyphenyl, Ethoxyphenyl, Hydroxyphenyl, Dihydroxyphenyl, Formyloxyphenyl, Acetyloxyphenyl, Propionyloxyphenyl, Butyryloxyphenyl, Benzyloxyphenyl, Trifluormethylphenyl, Fluorphenyl, Chlorphenyl, Bromphenyl, Difluorphenyl, Dichlorphenyl, Dibromphenyl in Frage.

Der Alkoxyrest in W kann Methoxy, Ethoxy, Propoxy, oder Butoxy sein.

Der Alkylthiorest in W kann Methylthio, Ethylthio, Propylthio oder Butylthio sein.

Für Halogen kommen Fluor, Chlor und Brom in Frage.

Gegenstand der Erfindung ist also die Umsetzung von Verbindungen der allgemeinen Formel II vorzugsweise in 50 % HF/Pyridin bzw. 50 % HF/Pyridin/H₂O in Gegenwart von tert.-Butylnitrit oder Natriumnitrit bei Temperaturen zwischen -50° und +25°C vorzugsweise aber bei Temperaturen zwischen -30° und -10°C zu Verbindungen der allgemeinen Formel I.

Der H₂O-Gehalt sollte zwischen zwischen 0% und 10 % liegen und das HF/Pyridin sollte zwischen 10 und 60 % HF enthalten.

Die benötigten Ausgangsmaterialien der allgemeinen Formel II für die Darstellung der 2-Fluorpurinderivate der allgemeinen Formel I lassen sich nach H. Vorbrüggen und K. Krolikiewicz, Liebigs Annalen 745 (1976) bzw. DOS 2441484 darstellen.

Falls erforderlich, können die funktionellen Gruppen in R¹ nach den dem Fachmann bekannten Methoden geschützt werden.

Die 5'-Phosphate können als freie Phosphorsäuren sowie auch als Salze, insbesondere als Natriumsalze, oder als Benzylester eingesetzt werden.

Bei Einsatz der Benzylester können diese nach dem Fachmann bekannten Methoden nach der 2-Fluorierung wieder gespalten werden.

Die Aufarbeitung der HF/Pyridin-Reaktionslösung erfolgt zweckmäßigenveise mit einer Suspension von überschüssigem feingepulvertem CaCO₃ in Eiswasser unter lebhaftem Rühren und Filtration des dabei entstehenden Calciumfluorides. Ferner kann man dem rohen Reaktionsgemisch überschüssiges Hexamethyldisiloxan unter heftigem Rühren zugeben, damit sich die Phasen zumindest etwas mischen, wobei bei Temperaturen zwischen -20°C und 24°C das flüchtige Trimethylsilylfluorid ( Kp 17°C) und Wasser entstehen. Das Trimethylsilylfluorid kann in einer Vorlage mit Lauge zu Alkalifluoriden und Hexamethyldisiloxan gespalten werden. Die Aufarbeitung mit Hexamethyldisiloxan ist insbesondere für die Umsetzung der 5'-Phosphate geeignet.

Nach der 2-Fluorierung werden, falls vorher eingeführt, die Schutzgruppen der funktionellen Gruppen in R¹ nach den dem Fachmann bekannten Methoden wieder gespalten.

Bei Verbindungen der allgemeinen Formel I mit R²=H kann auch nach der 2-Fluorierung nach dem Fachmann bekannten Methoden die Phosphatgruppe eingeführt werden.

Das 2-Fluoradenosin und seine N⁶-substituierten Analoga gemäß Formel I sind wertvolle Zwischenprodukte zur Darstellung von neuen therapeutisch wichtigen Nucleosiden und Nucleotiden.

So erhält man durch Schutz der 3',5'-alkoholischen Hydroxylgruppen in den Verbindungen der Formel I (R²= H ) z.B. mit 1,3-Dichloro-1,1,3,3-tetraisopropyl-disiloxan in Pyridin die geschützten Derivate, in denen sich die 2'-Hydroxylgruppe selektiv in das Triflat, das Nonaflat, Mesylat oder Tosylat überführen läßt. Anschließende Walden-Inversion durch Umsetzung mit Alkali-, Amin- oder Tetraalkylammoniumsalzen z.B. der Essigsäure oder Benzoesäure ergibt die geschützten Ara-Derivate. Nach Abspaltung der Schutzgruppen kann beispielsweise das so erhaltene Ara-2-fluoradenosin in 5'-Stellung selektiv zum als Ara-2-fluoroadenosin-5'-phosphat (Fludara) bekannten Leukämie-Therapeutikum phosphoryliert werden.

### REAGENZIEN

### 1) Herstellung von 50 % HF in Pyridin:

Zu 5 ml 70 % HF in Pyridin (Aldrich) werden bei -60°C langsam 2 ml abs. Pyridin zugetropft und auf -30°C erwärmt.

### 2) Dünnschichtchromatopgraphie:

Für die Dünnschichtchromatographie werden HPTLC-Nano-Kieselgelplatten No. 5628 der Firma Merck (Darmstadt) verwendet.

### 3) Kieselgel für die Säulenchromatographie:

Für die Säulenchromatographie wird Kieselgel No. 10757, das 40 % H₂O enthält, der Firma Merck (Darmstadt) verwendet.

### BEISPIELE

### Beispiel 1

### 2-Fluoradenosin

In 7 ml 50 % HF in Pyridin werden 1,13 g (4 mMol) feingepulvertes 2-Aminoadenosin bei -30 °C unter lebhaften Rühren suspendiert und anschließend 1,03 g 90 % tert.-Butylnitrit (= 9 mMol) innerhalb von 15 Minuten mit Hilfe einer Spritze langsam zugegeben, wobei eine leichte Gasentwicklung eintritt und das suspendierte 2-Aminoadenosin unter Gelbfärbung des Reaktionsgemisches in Lösung geht. Nach 30 Minuten Rühren bei -20°C → -25°C sind bei der Dünnschichtchromatographie (obere Phase von n-Butanol-Essigsäure-H₂O = 4 : 1 : 5) neben dem neu entstandenen 2-Fluoradenosin (R_{f} = 0,57) nur noch Spuren 2-Aminoadenosin (R_{f} = 0,4) sowie etwas Isoguanosin (R_{f} = 0,25) nachweisbar. Die Reaktionsmischung wird zu einer eiskalten Suspension von 50 g (0,5 Mol) Calciumcarbonat unter lebhaften Rühren unter CO₂ - Entwicklung zugetropft und die Suspension anschließend 30 Minuten bei 0°C gerührt. Den ungelösten Niederschlag von Calciumcarbonat und Calciumfluorid filtriert man anschließend über eine Schicht von ca. 20 g Celite und wäscht mit 150 ml einer Mischung von Pyridin-H₂O (1 : 9) nach. Nach Abdampfen des hellgelben Filtrats, bei 10 mm Vakuum, wird der partiell kristalline Rückstand (1,3 g) in 30 ml Methanol-H₂O (1 : 1) bei 40°C suspendiert, wobei geringe Mengen ungelöst bleiben, mit 10 g Kieselgel (40 % H₂O) versetzt und bei 10 mm Vakuum eingeengt.
Nach Aufgeben des die Substanz enthaltenen trockenen Kieselgels auf eine Säule von 15 g Kieselgel (40 % H₂O), die mit der oberen Phase des Gemisches von Essigester-Methylglycol-H₂O = 4 : 1 : 2 aufgezogen wurde, ergibt die Elution mit diesem Gemisch in den ersten 100 ml nur Spuren Substanz, die verworfen werden, während die nächsten 300 ml Eluat 0,760 g (66,7 %) reines 2-Fluoradenosin ergeben, das beim Erwärmen mit 5 ml H₂O auf dem Wasserbad kristallisiert, Schmpt. 237 - 242°C. Weitere Elution mit 50 ml liefert noch 18 mg leicht verunreinigtes 2-Fluoradenosin.

### Beispiel 2

### Darstellung von 2-Fluroadenosin

Wie in Beispiel 1 beschrieben, werden 1,13 g (4 mMol) feingepulvertes 2-Aminoadenosin bei -25°C in 7 ml 50 % HF in Pyridin suspendiert und unter Rühren innerhalb von 15 Minuten bei -25°C 0,55 g (8 mMol) Natriumnitrit portionsweise zugegeben. Nach 1 h bei -25 °C wird wie unter Beispiel 1 beschrieben mit CaCO₃ aufgearbeitet wobei 1,4 g Rohprodukt erhalten werden, die bei der Chromatographie an Kieselgel 0,708 g (62,1 %) reines, homogenes 2-Fluoradenosin liefern.

### Beispiel 3

### Darstellung von 2-Fluoradenosin

In einer Mischung von 7 ml 50 % HF-Pyridin und 0,35 ml H₂O werden bei -30°C 0,282 g (1 mMol) 2-Aminoadenosin suspendiert und innerhalb von 30 Minuten 1,2 ml (- 9 mol) tert.Butylnitrit langsam unter lebhaften Rühren bei -30°C zugetropft. Nach weiteren 30 Minuten bei -25°C → -30°C wird die Reaktionsmischung langsam in 120 ml 33 % wässrigen Ammoniak bei -10°C unter Rühren eingetropft und 1 h bei 0°C gerührt. Nach Zugabe von 7 g festem Calciumhydroxyd rührt man weitere 15 Minuten bei 0°C, filtriert und wäscht mit 40 ml H₂O. Das hellgelbe Filtrat wird vorsichtig bei ca. 25°C - 30°C auf ca. 15 ml eingeengt und CO₂ eingeleitet. Nach Abfiltrieren der geringen Menge Niederschlags und nach Waschen des Niederschlages mit 10 ml H₂O wird das Filtrat bei 10 mm Vakuum eingeengt und der gelblich-braune Rückstand (0,39 g) in 15 ml Methanol-H₂O (1 : 1) gelöst, mit 10 g Silicagel (40 % H₂O) versetzt und bei 10 mm Vakuum abgedampft. Nach Aufgabe des trockenen die Substanz enthaltenden Kieselgels, auf eine Säule von 15 g Kieselgel (40 % H₂O), die mit Isopropanol aufgezogen wurde, ergibt die Elution mit ca. 300 ml Isopropanol 0,261 g (≈ 91 %) rohes 2-Fluoradenosin, das noch wenig 2-Aminoadenosin sowie Isoguanosin enthält.

### Beispiel 4

### 2-Fluor-N⁶,N⁶-Pentamethylenadenosin

Wie in Beispiel 1 beschrieben werden 0,7 g (2 mMol) feingepulvertes 2-Amino-N⁶,N⁶-Pentanmethylenadenosin bei -25° C in 7 ml 50 % HF-Pyridin gelöst und 0,412 g (2 mMol) tert.Butylnitrit innerhalb von 5 min. unter Rühren zugegeben. Nach 30 min. bei -25° C wird wie unter Beispiel 1 angegeben, aufgearbeitet und die Celite-CaCO₃-CaF₂ Schicht mit 150 ml Methanol-H₂O (1:1) nachgewaschen. Abdampfen des Filtrats ergibt 0,89 g Rohprodukt, das in 20 ml Methanol gelöst und mit 15 g Silicagel versetzt und eingedampft wird. Diese die Substanz enthaltenen trockenen 15 g Silicagel werden auf eine Säule von weiteren 15 g Silicagel, das 40 % H₂O enthält gegeben und mit der oberen Phase von Essigester-Methylglycol-H₂O (4:1:2) entwickelt. Nach einem Vorlauf von 25 ml liefern die weiteren 125 ml Eluat 0,64 (90 %) hellgelbes kristallines 2-Fluor-N⁶,N⁶-Pentamethylenadenosin, das bei der Umkristallisation aus Essigester farblose analysenreines Produkt, Schmpt. 164° C ergibt.

### Beispiel 5

### Darstellung von 2-Fluorinosin

0,566 g (2 mMol) Guanosin-Dihydrat werden wie unter Beispiel 1 beschrieben in 7 ml HF/Pyridin gelöst und bei -25° C mit 0,412 g (2 mMol) tert.Butylnitrit innerhalb von 5 min. versetzt und anschließend noch 30 min. bei -30° C gerührt, aufgearbeitet und die Celite-CaCO₃-CaF₂ Schicht mit 150 ml Methanol-H₂O (1:1) nachgewaschen. Nach Abdampfen des Filtrats, Lösen des Rückstandes (0,9 g) in 10 ml H₂O, Zufügen von 15 g Silicagel und Abdampfen aus Rotationsverdampfer wird trockenes, die Substanz enthaltenes Silicagel, erhalten. Nach Aufgabe des trockenen Silicagel auf eine mit der oberen Phase von n-Butanol-Essigsäure H₂O (4:1:5) hergestellten Säule von weiteren 15 g Silicagel, den 40 % H₂O enthält, ergeben die ersten 150 ml Eluat 0,52g ( 90,9%) farblos kristallines 2-Fluorinosin .

### Beispiel 6

10 g (35,43 mmol) 2-Aminoadenosin werden in einer 250 ml Polyethylenflasche bei -5 °C mit 50 ml 56 % HF-Pyridin-Lösung versetzt und die Suspension langsam unter Rühren auf 12 °C erwärmt, wobei eine klare Lösung entsteht. anschließend wird auf -16 °C Innentemperatur gekühlt und eine Lösung von 3,4 g (40 mmol) KNO₂ in 2 ml Wasser innerhalb von 20 Minuten bei -16 bis -7 °C Innentemperatur unter Rühren zugetropft. Nach weiteren 30 Minuten Rühren bei -7 ° bis -10 °C wird die Reaktionsmischung noch 3 Stunden bei +30 °C belassen, auf -35 °C gekühlt und mit 41,5 ml absolutem Pyridin langsam und vorsichtig innerhalb von 40 Minuten verdünnt, wobei ein feiner, schmieriger Niederschlag ausfällt. Anschließend wird 50,2 ml Acetanhydrid bei -5 °C bis +12 °C innerhalb von 30 Minuten vorsichtig zugetropft, wobei unter Schäumen CH₃COF entweicht. Nach ca. 4 Stunden bei +18 °C bis +22 °C gibt man die Reaktionsmischung langsam zu einer Lösung von 30,92 g (0,5 Mol) Borsäure in 600 ml H₂O rührt noch 30 Minuten und filtriert.

Die Kristalle wäscht man portionsweise mit 80 ml H₂O sowie 80 ml Methanol. Die leicht gefärbten Kristalle ergeben nach Trocknen im Vakuum bei +60 °C 10,5 g 2-Fluoradenosin-2',3',5'-tri-O-acetat vom Schmelzpunkt 201,9 °C.

Nach Einengen des Filtrats auf 400 ml und wiederholter Extraktion mit insgesamt 1 Liter Essigester, wird die Essigesterlösung mit NaHCO₃ und NaCl-Lösung gewaschen, über Na₂SO₄ getrocknet und eingeengt, wobei 1,5 g teilweise kristalliner Rückstand erhalten wird (Rohausbeute 12 g = 83,3 °C).

Umkristallisation aus Essigester-Ethanol (4:1) liefert 10,61 g (72,8 %) reines 2-Fluoradenosin-2',3',5',-tri-O-acetat vom Schmelzpunkt 203,9 °C.

## Patentansprüche

1. Verfahren zur Herstellung von 2-Fluorpurin-Derivaten der allgemeinen Formel I, 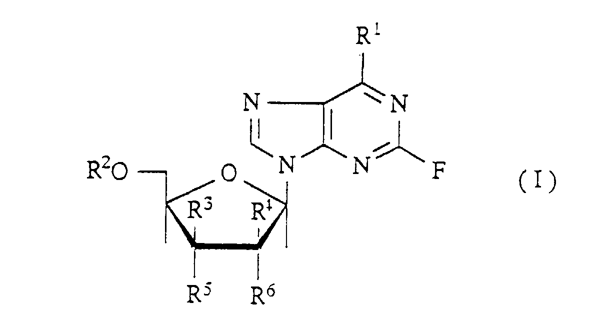 worin
R¹ OH, NH₂, NR⁷R⁸ oder NR⁹R¹⁰, und
R² Wasserstoff oder 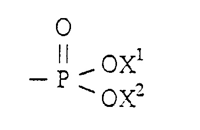 bedeuten, wobei
X¹,X² unabhängig voneinander Wasserstoff, Li, Na, K oder Benzyl darstellen,
R³,R⁴,R⁵,R⁶ unabhängig voneinander Wasserstoff oder OH, mit der Maßgabe, daß niemals zwei Hydroxyreste an demselben Kohlenstoffatom stehen,
R⁷,R⁸ unabhängig voneinander Wasserstoff, C₁-C₄ Alkyl, C₇-C₈ Aralkyl, einen heterocyclischen Rest, C₃-C₇ Cycloalkyl, oder R⁷ und R⁸ gemeinsam eine C₄-C₇ Ring, der gegebenenfalls 1-3 weitere Heteroatome enthält, darstellen, oder wenn R⁷ Wasserstoff bedeutet, R⁸ OH, oder NH₂ darstellen kann,
R⁹ Wasserstoff oder C₁-C₄ Alkyl,
R¹⁰ -(CH₂)ₙ-R¹¹, 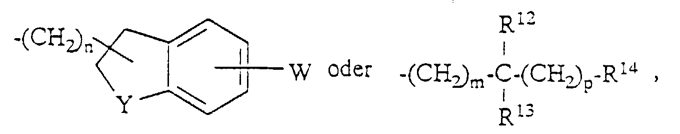
m,n,p unabhängig voneinander 0,1 oder 2,
R¹¹ gegebenenfalls durch C₁-C₄ Alkyl substituierte Reste C₃-C₇ Cycloalkyl, Cyclohexenyl, Bicycloheptyl oder Bicycloheptenyl, und
Y Stickstoff, Sauerstoff, Schwefel, Methylen, -CH₂Z- oder -ZCH₂-, bedeuten, wobei Z Stickstoff, Sauerstoff, oder Schwefel darstellt,
R¹²,R¹³ unabhängig voneinander Wasserstoff, OH, Phenyl oder C₁-C₄ Alkyl,
R¹⁴ Wasserstoff, OH, C₁-C₄ Alkyl, Phenyl oder gegebenenfalls durch ein bis drei der folgenden Reste C₁-C₄ Alkyl, C₁-C₄ Alkoxy, OH, C₁-C₄ Alkanoyloxy, Benzyloxy, Trifluormethyl, oder Halogen substituiertes Phenyl,
W Wasserstoff, C₁-C₄ Alkoxy, C₁-C₄Alkylthio, Halogen oder gegebenenfalls durch OH, C₁-C₄Alkoxy, CN, COOR¹⁵ oder CONHR¹⁶ substituiertes C₁-C₄ Alkyl,
R¹⁵ Wasserstoff oder C₁-C₄ Alkyl,
R¹⁶ Wasserstoff oder C₁-C₄ Alkyl,
bedeuten,
dadurch gekennzeichnet, daß man ein 2-Aminopurin-Derivat der allgemeinen Formel II 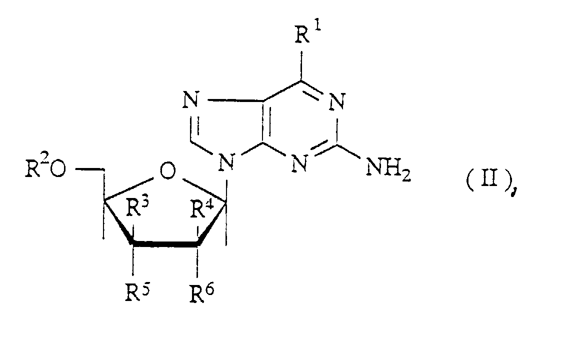 worin R¹, R², R³, R⁴, R⁵ und R⁶ die oben angegebenen Bedeutungen haben, mit 50% HF/Pyridin oder 50% HF/Pyridin/H₂O in Gegenwart von einem Nitrit der allgemeinen Formel III
R¹⁷ONO (III),
worin R¹⁷ tert.-Butyl, Li, Na, oder K bedeutet,
bei einer Temperatur zwischen -50°C und +25°C umsetzt und gegebenenfalls, wenn R² Wasserstoff bedeutet, nachträglich in das 5'-Phosphat überführt.

## Claims

1. Process for the preparation of 2-fluoropurine derivatives of the general formula I 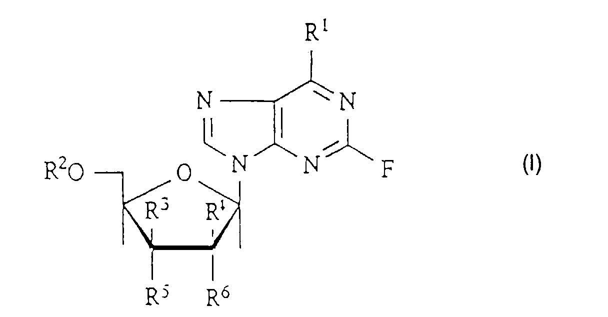 wherein
R¹ represents OH, NH₂, NR⁷R⁸ or NR⁹R¹⁰, and
R² represents hydrogen or 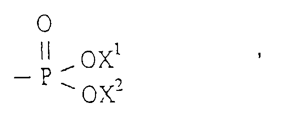 wherein
X¹ and X² each independently of the other represents hydrogen, Li, Na, K or benzyl,
R³, R⁴, R⁵ and R⁶ each independently of the others represents hydrogen or OH, with the proviso that there are never two hydroxy radicals on the same carbon atom,
R⁷ and R⁸ each independently of the other represents hydrogen, C₁-C₄alkyl, C₇-C₈aralkyl, a heterocyclic radical or C₃-C₇cycloalkyl, or R⁷ and R⁸ together form a C₄-C₇ ring that optionally contains from 1 to 3 further hetero atoms, or, when R⁷ represents hydrogen, R⁸ may represent OH or NH₂,
R⁹ represents hydrogen or C₁-C₄alkyl,
R¹⁰ represents -(CH₂)ₙ-R¹¹, 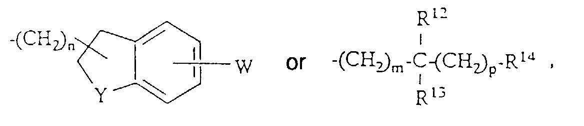
m, n and p are each independently of the others 0,1 or 2,
R¹¹ represents a C₃-C₇cycloalkyl, cyclohexenyl, bicycloheptyl or bicycloheptenyl radical, each of which is optionally substituted by C₁-C₄alkyl, and
Y represents nitrogen, oxygen, sulphur, methylene, -CH₂Z- or -ZCH₂- in which Z represents nitrogen, oxygen or sulphur,
R¹² and R¹³ each independently of the other represents hydrogen, OH, phenyl or C₁-C₄alkyl,
R¹⁴ represents hydrogen, OH, C₁-C₄alkyl, phenyl or phenyl that is optionally substituted by from 1 to 3 of the following radicals C₁-C₄alkyl, C₁-C₄alkoxy, OH, C₁-C₄alkanoyloxy, benzyloxy, trifluoromethyl, or halogen,
W represents hydrogen, C₁-C₄alkoxy, C₁-C₄alkylthio, halogen or C₁-C₄alkyl that is optionally substituted by OH, C₁-C₄alkoxy, CN, COOR¹⁵ or by CONH R¹⁶,
R¹⁵ represents hydrogen or C₁-C₄alkyl, and
R¹⁶ represents hydrogen or C₁-C₄alkyl,
characterised in that a 2-aminopurine derivative of the general formula II 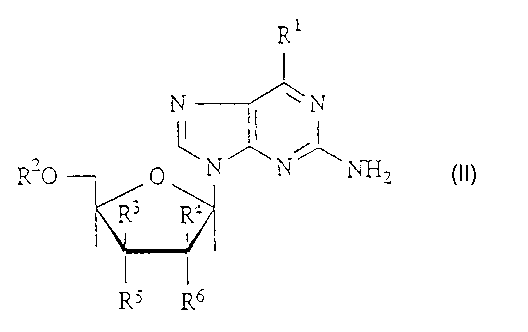 wherein R¹, R², R³, R⁴, R⁵ and R⁶ have the meanings given above,
is reacted with 50% HF/pyridine or 50% HF/pyridine/H₂O in the presence of a nitrite of the general formula III
R¹⁷ONO (III)
wherein R¹⁷ represents tert-butyl, Li, Na or K,
at a temperature of from -50°C to +25°C and, if desired, when R² represents hydrogen, is subsequently converted into the 5'-phosphate.

## Revendications

1. Procédé de fabrication de dérivés de la 2-fluoropurine de la formule générale I, 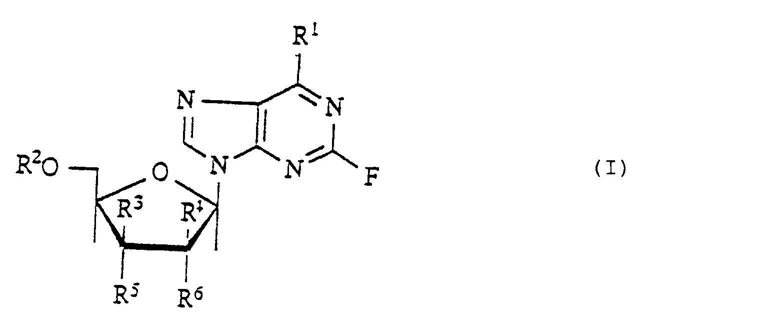 dans laquelle
R¹ est OH, NH₂, NR⁷R⁸ ou NR⁹R¹⁰, et
R² est l'hydrogène ou 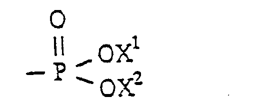
X¹, X² représentant, indépendamment l'un de l'autre, l'hydrogène, Li, Na, K ou benzyle,
R³, R⁴, R⁵, R⁶ sont, indépendamment les uns des autres, l'hydrogène ou OH, à la condition qu'il n'y ait jamais deux restes hydroxy sur le même atome de carbone,
R⁷, R⁸ sont, indépendamment l'un de l'autre, l'hydrogène, alkyle en C₁-C₄, aralkyle en C₇-C₈, un reste hétérocyclique, cycloalkyle en C₃-C₇, ou bien R⁷ et R⁸ ensemble représentent un cycle en C₄-C₇ contenant le cas échéant 1 à 3 autres hétéroatomes, ou bien, lorsque R⁷ est l'hydrogène, R⁸ peut être OH ou NH₂,
R⁹ est l'hydrogène ou alkyle en C₁-C₄,
R¹⁰ est -(CH₂)ₙ-R¹¹, 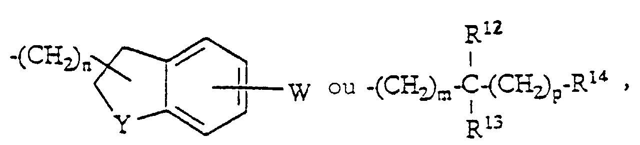
m,n,p sont, indépendamment les uns des autres, égaux à 0, 1 ou 2,
R¹¹ est un reste bicyloheptyle, bicycloheptényle, cyclohexényle ou cycloalkyle en C₃-C₇, le cas échéant substitué par alkyle en C₁-C₄,
Y est un atome d'azote, d'oxygène, de soufre, méthylène, -CH₂Z- ou -ZCH₂-,
Z représentant un atome d'azote, d'oxygène ou de soufre,
R¹², R¹³ sont, indépendamment l'un de l'autre, l'hydrogène, OH, phényle ou alkyle en C₁-C₄,
R¹⁴ est l'hydrogène, OH, alkyle en C₁-C₄, phényle ou bien phényle le cas échéant substitué par un à trois des restes suivants: alkyle en C₁-C₄, alcoxy en C₁-C₄, OH, alcanoyloxy en C₁-C₄, benzyloxy, trifluorométhyle ou halogène,
W est l'hydrogène, alcoxy en C₁-C₄, alkylthio en C₁-C₄, halogène ou alkyle le cas échéant substitué par OH, alcoxy en C₁-C₄, CN, COOR¹⁵ ou CONHR¹⁶,
R¹⁵ est l'hydrogène ou alkyle en C₁-C₄,
R¹⁶ est l'hydrogène ou alkyle en C₁-C₄,
caractérisé en ce que l'on fait réagir à une température comprise entre -50°C et +25°C un dérivé de la 2-amino-purine de formule générale II 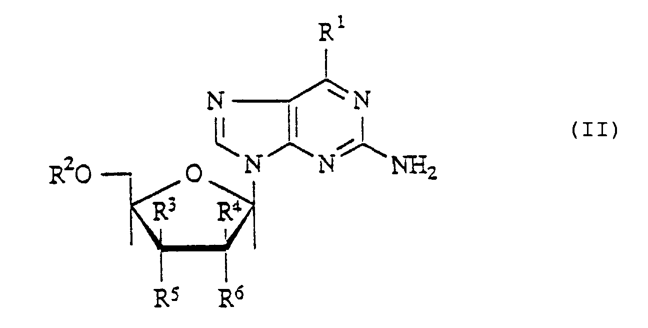 dans laquelle R¹, R², R³, R⁴, R⁵ et R⁶ ont les significations indiquées ci-dessus, dans du HF/pyridine à 50% ou du HF/pyridine/H₂O à 50% en présence d'un nitrite de formule générale III
R¹⁷ONO (III)
dans laquelle R¹⁷ est tert-butyle, Li, Na ou K, et le cas échéant, lorsque R² est l'hydrogène, on le convertit ensuite en 5' phosphate.
